# EUROPEAN PATENT SPECIFICATION

(11) **EP 2 853 257 B1**
(45) Date of publication and mention of the grant of the patent: **02.11.2022**
(21) Application number: 14184462.1
(22) Date of filing: 11.09.2014
(51) Int. Cl.: A61K 9/00, A61K 9/20, A61K 31/522, A61P 3/10

(54) **Pharmaceutical formulations of linagliptin**
Pharmazeutische Formulierungen von Linagliptin
Formulations pharmaceutiques de linagliptine

(30) Priority: 12.09.2013 TR 201310724
(43) Date of publication of application: 01.04.2015
(60) Divisional application: 22151084.5
(73) Proprietor: Sanovel Ilac Sanayi ve Ticaret A.S., 34460 Istanbul (TR)
(72) Inventor: Türkyilmaz, Ali, 34460 Istanbul (TR); Turp, Ali Hasan, 34460 Istanbul (TR); Saydam, Mehtap, 34460 Istanbul (TR)
(74) Representative: Sevinç, Erkan

(56) References cited:
- EP-B1- 2 023 902
- WO-A1-2010/092124
- WO-A2-2013/062902
- Anonymous: "IMCD | Croscarmellose Sodium", , 6 September 2021 (2021-09-06), XP055838148, Retrieved from the Internet: URL:https://www.imcdus.com/en-us/products/ croscarmellose-sodium [retrieved on 2021-09-06]
- -: "Croscarmellose sodium", , 6 September 2021 (2021-09-06), XP055838260, Retrieved from the Internet: URL:https://de.wikipedia.org/wiki/Croscarm ellose-Natrium [retrieved on 2021-09-06]

## Description

### Field of Invention

A pharmaceutical formulation for therapeutically effective amount of linagliptin comprising croscarmellose sodium and at least one other pharmaceutically acceptable excipient.

### Background of Invention

Linagliptin is used for type 2 or non-insulin dependent diabetes. It is a selective, orally administered, xanthine based dipeptidyl peptidase-4 (DPP-4) inhibitor used as an adjunct to diet and exercise to improve glycemic control. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Linagliptin works by binding to DPP-4 and preventing it from breaking down the GLP-1 and GIP. This increases the levels of these hormones in the body and so increases their effect on controlling blood sugar.

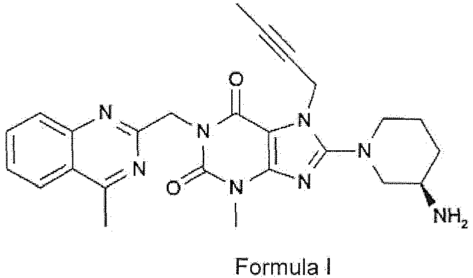

Its chemical name is 8-[(3R)-3-aminopiperidin-1-yl]-7-but-2-yn-1-yl)-3-methyl-1-[(4-methylquinazolin-2-yl)methyl]-3,7-dihydro-1H-purine-2,6-dione and its chemical structure is shown in the Formula I.

There is Linagliptin coated tablet formulation license in the US and EU under the brand name TRADJENTA^{®} by Boehringer Ingelhem International. TRADJENTA^{®} includes mannitol, prejelatinized starch, maize starch, copovidone, magnesium stearate in the tablet core and hypromellose, titanium dioxide, talc, macrogol 6000 and red iron oxide in the film coating.

It is used once daily and can be used either alone or in combination with insulin or other glycemic agents. The currently commercially available dose of Linagliptin is 5 mg and due to the low active substance content in the final formulation, the active substance is milled in order to ensure an adequate content uniformity. Contrary to commercially available dose of Linagliptin, in this present invention, to obtain desired content uniformity, croscarmellose sodium was used as a disintegrant.

EP 2 023 902 B1 provides pharmaceutical formulations comprising DPP-4 inhibitors included Linagliptin. It is indicated that DPP-4 inhibitors with primary amine group shows incompatibilities, degradation problems or extraction problems with excipients such as microcrystalline cellulose, sodium starch glycolate, croscarmellose sodium, tartaric acid, citric acid, glucose, fructose, saccharose, lactose, maltodextrines. Linagliptin has also a primary amine group on its chemical structure. In solid dosage forms, it may react with many excipients or impurities of excipients, although linagliptin itself is very stable. Thus, aforementioned patent provides Linagliptin formulations which do not comprise above excipients included croscarmellose sodium.

WO 2013/062902 A2 relates to pharmaceutical formulations comprising a dipeptidiyl peptidase-4 inhibitor and atorvastatin or a pharmaceutically acceptable salt thereof, methods of preparing such pharmaceutical compositions, and methods of treating Type 2 diabetes with such pharmaceutical compositions. Linagliptin is mentioned within suitable DPP-IV inhibitors. What is defined as a technical problem in WO 2013/062902 A2 is stability related problems; accordingly bilayer tablets in which DPP-IV inhibitor and atorvastatin are present in different layers. Bilayer tablet may also comprise croscarmellose sodium in both layers wherein first layer may comprise 0.1-10% of croscarmellose sodium and the second layer may comprise 0.1-20% of croscarmellose sodium as disintegrant. DPP-IV inhibitor is formulated to be in the first layer; while atorvastatin is present in the second layer.

WO2010092124 A1 relates to pharmaceutical compositions comprising linagliptin as a first active pharmaceutical ingredients. WO2010092124 A1 also discloses a pharmaceutical composition comprising linagliptin and an SGLT2 inhibitor. WO2010092124 A1 addresses incompatibility problems encountered in formulations comprising DPP-IV inhibitors such as linagliptin and a number of customary excipients. According to the passage on page 3, lines 8-10 of WO2010092124 A1; the main object is to minimize degradation of linagliptin and enable a good shelf life. In addition to this, content uniformity, disintegration time, dissolution and bioavailability are defined. Based on the disclosure on page 5, lines 18-22 of WO2010092124 A1; linagliptin with a particle size distribution of X90<200 mcm shows improved dissolution profile, high content uniformity and good bioavailability.

In this invention, croscarmellose sodium has been used as a disintegrant to achieve required content uniformity in the solid dosage form of linagliptin. To overcome stability problem if may arise from croscarmellose, at least one other pharmaceutically acceptable excipient has been used.

### Description of the invention

According to the present invention the pharmaceutical formulation of linagliptin comprises
a. 3.5 - 60.0 % by weight of linagliptin
b. 10.0 - 20.0 % by weight of croscarmellose sodium
c. 30.0 - 50.0 % by weight of dibasic calcium phosphate
d. 0.25 -2.0 % by weight of sodium stearyl fumarate
e. 0.1 - 25.0 % by weight of pullulan
f. 0.1 - 3 % by weight of silicon dioxide
g. 0.2 - 10 % by weight of coating
by weight of total formulation.

Linagliptin is present in an amount of 3.5 to 60 % and more preferably 10 to 40 % by weight of total formulation.

It is known that obtaining an adequate content uniformity is an important issue for pharmaceutical solid dosage forms during the process, because it is difficult to disintegrate active ingredients homogenously, especially if they have low doses of active agent. Moreover, inadequate content uniformity results undesired side effects and low bioavailability during the therapy. In this invention, to obtain desired content uniformity, croscarmellose sodium was used as a disintegrant. Without a milling process of linagliptin as in state of art, desired content uniformity has been achieved by using croscarmellose sodium.

The amount of croscarmellose sodium is in the range of 10 to 20 % by weight of total formulation.

According to this invention, the final dosage form of the pharmaceutical formulation of linagliptin has a content uniformity of less than 3.0 % RSD (Relative Standard Deviation), preferably it has a content uniformity of less than 2.0 % RSD. Herein, content uniformity test is determined by calculating RSD (Relative Standard Deviation) value of 10 tablets and RSD value is determined as described in pharmacopoeia USP31-NS26S2, chapter 905.

According to the present invention, suitable filler is dibasic calcium phosphate.

In general, DPP-4 inhibitors are not very stable compounds. Especially, in solid dosage forms, amine group containing DPP-4 inhibitors like linagliptin may react with many excipients or impurities of excipients. In prior art, croscarmellose sodium has been indicated as a reactive substance with amine group containing DPP-4 inhibitors. In this invention, it has been surprisingly found that using dibasic calcium phosphate ensures the stability of linagliptin in a solid dosage formulation. Furthermore, dibasic calcium phosphate gives good flow and compaction properties to the formulation due to its bulk density.

The stability assay is performed by HPLC at a wavelength of 225nm at 45 °C in the presence of phosphate buffer.

According to the present invention, the amount of dibasic calcium phosphate is in the range of 30 to 50 % by weight of total formulation.

According to the present invention, suitable binder is pullulan.

According to the present invention, suitable lubricant is sodium stearyl fumarate,.

In an embodiment of the invention the linagliptin solid dosage formulation does not comprise magnesium stearate in contrast to prior art. Magnesium stearate is a metal derivative lubricant. It has anti-adhesive and flow enhancement properties by ensuring uniformity of tablets. Besides this advantageous, its hydrophobic properties has disadvantages on the process such as prolonged mixing. Because of this increase in time, lubricant forms a hydrophobic film around the active agent and retards the dissolution by retarding wetting of active agent as well as decreases the compatibility of the powder mixture.

In this invention, sodium stearyl fumarate is selected as a suitable lubricant in tableting. Due to its hydrophilic properties, formulation does not have the disadvantages of magnesium stearate in respect of tablet compatibility, disintegration and dissolution.

In an embodiment of the invention, the pharmaceutical formulation comprises sodium stearyl fumarate and said formulation does not comprise magnesium stearate.

The amount of sodium stearyl fumarate is present in the range of 0.25 to 2.0 % by weight of total formulation.

The further advantage of the formulation with croscarmellose and dibasic calcium phosphate is to further improve disintegration and dissolution of linagliptin by using sodium stearyl fumarate without using magnesium stearate that cause undesirable dissolution profile. According to the present invention, the formulation wherein 75 to 90 % of the total amount of linagliptin by weight relative to the total weight of said formulation is dissolved in 45 minutes, preferably 30 minutes, more preferably in 15 minutes when measured in 900mL of 0.1N HCl at 50 rpm named as USP Apparatus I (Basket).

Suitable glidant is colloidal silicon dioxide.

Coating may also preferably be used for moisture protection. It can be selected from the group comprising Polyvinyl alcohol-polyethylene glycol copolymers (Kollicoat IR), polyvinyl alcohol or copolymers or mixtures thereof (Opadry AMB), Ethylcellulose Dispersions (Surelease), Kerry-HPC, polyethylene glycol, polyvinylprolidone, polyvinylprolidone-vinyl acetate copolymer(PVP-VA), and all kinds of OpadryTM, as well as pigments, dyes, titanium dioxide and iron oxide, talk and polymethylmetacrylate copolymers (Eudragit).

In this present invention, to achieve desired content uniformity and stability with an improved process, these formulations have been designed, comprising the following:
a. 3.5 - 60.0 % by weight of linagliptin
b. 10.0 - 20.0 % by weight of croscarmellose sodium
c. 30.0 - 50.0 % by weight of dibasic calcium phosphate
d. 0.25 -2.0 % by weight of sodium stearyl fumarate
e. 0.1 - 25.0 % by weight of pullulan
f. 0.1 - 3 % by weight of silicon dioxide
g. 0.2 - 10 % by weight of coating

### Example 1: Wet Granulation

| **ingredients** | **Amount (%)** |
|---|---|
| linagliptin | 3.5 - 60.0 |
| dibasic calcium phosphate | 10.0 - 75.0 |
| croscarmellose sodium | 6.0 - 25.0 |
| pullulan | 0.1 - 25.0 |
| silicon dioxide | 0.1 - 0.2 |
| sodium stearyl fumarate | 0.1 - 5.0 |
| coating | 0.2 - 5.0 |

The production of the formulation is carried out as follows: Linagliptin, croscarmellose sodium and dibasic calcium phosphate are mixed and granulated with alcoholic/hydroalcoholic pullulan solution. Then, granules are dried and sieved. They are mixed with silicon dioxide and sodium stearyl fumarate respectively, and then pressed into tablets. Pressed tablets are preferably coated with PVA (polivinil alkol) based coatings such as Opadry AMB/ Kollicoat IR.

### Example 2: Pellet - Capsule

| **ingredients** | **Amount (%)** |
|---|---|
| linagliptin | 3.5 - 60.0 |
| dibasic calcium phosphate | 10.0 - 75.0 |
| croscarmellose sodium | 6 - 25.0 |
| pullulan | 0.1 -25.0 |
| silicon dioxide | 0.1 - 0.2 |
| sodium stearyl fumarate | 0.1 - 5.0 |
| coating | 0.2 - 5.0 |

The production of the formulation is carried out as follows: Linagliptin, silicon dioxide, croscarmellose sodium and dibasic calcium phosphate are mixed and granulated with alcoholic/hydroalcoholic pullulan solution. Granules are passed through the extruder and pellets are obtained by spheronization. Pellets are mixed with sodium stearyl fumarate and filled into capsules or pressed into tablets. Pressed tablets are preferably coated with PVA (polivinil alkol) based coatings such as Opadry AMB/ Kollicoat IR.

### Example 3: Sugar Pellet - capsule

| **ingredients** | **Amount (%)** |
|---|---|
| linagliptin | 3.5 - 60.0 |
| dibasic calcium phosphate | 10.0 - 75.0 |
| croscarmellose sodium | 6 - 25.0 |
| pullulan | 0.1 - 25.0 |
| silicon dioxide | 0.1 - 0.2 |
| sodium stearyl fumarate | 0.1 - 5.0 |
| Sugar pellet | 5 - 90 |
| coating | 0.2 - 5.0 |

The production of the formulation is carried out as follows: The alcoholic/hydroalcoholic solution of Linagliptin with pullulan is prepared. Sugar pellets are coated with this solution. Pellets are mixed with croscarmellose sodium, dibasic calcium phosphate, silicon dioxide and sodium stearyl fumarate, respectively. Then, the mixture filled into capsules or pressed into tablets. Pressed tablets are preferably coated with PVA (polyvinyl alcohol) based coatings such as Opadry AMB/ Kollicoat IR.

With this invention, a pharmaceutical formulation comprising linagliptin is achieved which is eliminating disintegration, stability and process related problems and bringing additional advantages.

## Claims

1. A pharmaceutical formulation of linagliptin comprising
a. 3.5 - 60.0 % by weight of linagliptin
b. 10.0 - 20.0 % by weight of croscarmellose sodium
c. 30.0 - 50.0 % by weight of dibasic calcium phosphate
d. 0.25 - 2.0 % by weight of sodium stearyl fumarate
e. 0.1 - 25.0 % by weight of pullulan
f. 0.1 - 3 % by weight of silicon dioxide
g. 0.2 - 10 % by weight of coating
by weight of total formulation.

2. The pharmaceutical formulation according to claim 1, wherein linagliptin is present in an amount of 10 to 40 % by weight of total formulation.

## Patentansprüche

1. Pharmazeutische Formulierung von Linagliptin mit
a. 3,5 - 60,0 Gew.-% Linagliptin
b. 10,0 - 20,0 Gew.-% Croscarmellose-Natrium
c. 30,0 - 50,0 Gew.-% dibasisches Calciumphosphat
d. 0,25 - 2,0 Gew.-% Natriumstearylfumarat
e. 0,1 - 25,0 Gew.-% Pullulan
f. 0,1 - 3 Gew.-% Siliziumdioxid
g. 0,2 - 10 Gew.-% Überzug
bezogen auf das Gewicht der gesamten Formulierung.

2. Pharmazeutische Formulierung nach Anspruch 1, wobei Linagliptin in einer Menge von 10 - 40 Gew.-% der gesamten Formulierung vorliegt.

## Revendications

1. - Formulation pharmaceutique de linagliptine comprenant
a. 3,5 - 60,0 % en poids de linagliptine ;
b. 10 - 20,0 % en poids de croscarmellose sodique ;
c. 30,0 - 50,0 % en poids de phosphate de calcium dibasique ;
d. 0,25 - 2,0 % en poids de fumarate de stéaryle sodique ;
e. 0,1 - 25,0 % en poids de pullulane ;
f. 0,1 - 3 % en poids de dioxyde de silicium ;
g. 0,2 - 10 % en poids d'enrobage
en poids de la formulation totale.

2. - Formulation pharmaceutique selon la revendication 1, dans laquelle la linagliptine est présente dans une quantité de 10 à 40 % en poids de la formulation totale.
